# EUROPEAN PATENT SPECIFICATION

(11) **EP 1 218 402 B1**
(45) Date of publication and mention of the grant of the patent: **26.05.2004**
(21) Application number: 99963658.2
(22) Date of filing: 09.09.1999
(51) Int. Cl.: C07J 63/00, A61K 31/575

(54) **NOVEL BETULINIC ACID DERIVATIVES HAVING ANTIANGIOGENIC ACTIVITY, PROCESSES FOR PRODUCING SUCH DERIVATIVES AND THEIR USE FOR TREATING TUMOR ASSOCIATED ANGIOGENESIS**
BETULINSÄURE-DERIVATE MIT ANTIANGIOGENER WIRKUNG, VERFAHREN ZUR HERSTELLUNG SOLCHER DERIVATE UND DEREN VERWENDUNG FÜR DIE BEHANDLUNG TUMORASSOZIIERTER ANGIOGENESE
NOUVEAUX DERIVES D'ACIDE BETULINIQUE PRESENTANT UNE ACTIVITE ANTIANGIOGENESE, PROCEDES DE PRODUCTION DE CES DERIVES ET LEUR UTILISATION POUR TRAITER UNE TUMEUR ASSOCIEE A L'ANGIOGENESE

(43) Date of publication of application: 03.07.2002
(73) Proprietor: Dabur Research Foundation, Sahibabad, Ghaziabad, 201 010 (IN)
(72) Inventor: JAGGI, manu W-5/40 DLF Qutab Enclave Phase III, Haryana (IN); RAMADOSS, Sunder 52A DDA Flats, Haus Khas New Delhi 110 016 (IN); RAJENDRAN, Praveen 801 Sumeru Kaushambi, Uttar Pradesh (IN); AHMED SIDDIQUI, Mohammad, Jamshed, Patpargunj Delhi 110 092 (IN)
(74) Representative: Goddar, Heinz J., Dr.
(86) International application number: PCT/IN1999/000043
(87) International publication number: WO 2001/018029

(56) References cited:
- EP-A1- 0 542 622
- WO-A1-93/10142
- WO-A1-96/39033
- WO-A1-98/51293
- WO-A2-98/51294
- GB-A- 1 415 601
- CHEMICAL ABSTRACTS, vol. 128, no. 12, 23 March 1998, Columbus, Ohio, US; abstract no. 145138X, HITOSHI ET AL.: 'Lupenes as improvers for heterogenous skin and cosmetics containing the improvers for treatment of skin darkening and aging' page 960; XP002909878 & JP 1 017 431 A 20 January 1989
- CHEMICAL ABSTRACTS, vol. 129, no. 11, 14 September 1998, Columbus, Ohio, US; abstract no. 132518S, JAGADEESH ET AL.: 'Tobacco caterpillar antifeedant from the gotti sterm wood triterpene betalinic acid' page 218; XP002909879 & J. AGRIC. FOOD CHEM. vol. 46, no. 7, 1998, pages 2797 - 2799
- CHEMICAL ABSTRACTS, vol. 120, no. 23, 06 June 1994, Columbus, Ohio, US; abstract no. 289424V, FUJIOKA ET AL.: 'Anti-AIDS agents. 11. Betulinic acid and platanic acid as anti-HIV principles from Syzigium claviforum and the anti-HIV activity of structurally related triterpenoids' page 25; XP002909880 & J. NAT. PROD. vol. 57, no. 2, 1994, pages 243 - 247
- CHEMICAL ABSTRACTS, vol. 120, no. 21, 23 May 1994, Columbus, Ohio, US; abstract no. 270889D, PRADHAN ET AL.: 'Studies on reactions of 2-bromo-3-ketotriterpenoids: Part III- Reduction of 2 alpha-bromo and 2,2-dibromo derivatives of lupanone and methyldihydrobetulonate with lithium aluminum hydride and sodium borohydride' page 1122; XP002909881 & IND. J. CHEM. SECT. B vol. 32(B), no. 11, 1993, pages 1178 - 1180
- CHEMICAL ABSTRACTS, vol. 77, no. 21, 20 November 1972, Columbus, Ohio, US; abstract no. 140351E, WRZECIONO ET AL.: 'Triterpene nitrogenous derivatives VI 3- and 28-aminolupane derivatives III' page 475; XP002909882 & ROCZ. CHEM. vol. 46, no. 7/8, 1972, pages 1285 - 1293

## Description

### Field of Invention

This invention relates to novel betulinic acid derivatives and a composition containing betulinic acid derivatives, processes for preparation of such betulinic acid derivatives. This invention also relates to the use of the novel betulinic acid derivatives to manufacture a composition useful for inhibiting and/or preventing tumor associated angiogenesis, more specifically angiogenesis associated with prostate, lung, ovary and colon cancers.

### Background of the Invention

Betulinic acid is a pentacyclic triterpene. It can be derived from several natural (botanical) sources. It can also be chemically derived from betulin, a substance found in abundance in the outer bark of white birch trees (*Betula alba*). Betulinic acid has been found to selectively kill human melanoma cells (Nature Medicine, Vol.1(10),1995, WO 96/29068). The cytotoxic potential of betulinic acid was tested using three human melanoma cell lines, Mel-1, Mel-2, and Mel-4. The growth of all the cell lines was inhibited significantly by treatment with betulinic acid. The effectiveness of betulinic acid against melanoma cancer cells was also tested using athymic mice. It seems to work by inducing apoptosis in cancer cells.

The anti-cancer activity of betulinic acid and some of its derivatives has also been demonstrated using mouse sarcoma 180 cells implanted s.c. in nude mouse (JP 87,301,580), inhibition of growth of p388 lymphocytic leukemia cells in vitro (Choi.Y-H et al., Planta Medica Vol.XLVII,511-513,1988) and inhibiting growth of cancer cells, particularly by inhibiting ornithine decarboxylase (Yasukawa, K et al. Oncology **48**:72-76, 1991; WO 95/04526).

Recently, the applicants reported anti-leukemia and anti-lymphoma activity and anti-prostate, anti-lung and anti-ovarian cancer activity of betulinic acid and its derivatives with ED₅₀ values less than 4.0 µg/ml. (US Application Number 09/040,856 filed on March 18, 1998 and US Application Number 09/251,309 filed on February 17, 1999). Further, antiangiogenic activity of betulinic acid and its derivatives was recently reported by the applicants in US Application number 09/166,809 filed on October 06, 1998 wherein betulinic acid and its derivatives were shown to inhibit the formation of tube-like-structures (TLS) of endothelial cells when grown on Matrigel coated surface. The endothelial cell anti-proliferative activity along with anti-TLS activity was shown to suggest the anti-angiogenic activity of betulinic acid derivatives.

Anderson et al (WO 95/04526) disclose that for certain cancers to spread throughout a patients' body, a process termed metastasis, cell-cell adhesion must take place. Specifically, cancer cells must migrate from their site of origin and gain access to blood vessel to facilitate colonization at distant sites. Certain cancer cells are known to adhere to E-Selectin via E-Selectin ligands on their cell surface and this event is one component of the metastasis process. Betulinic acid and its derivatives interfere with Selectin binding. Betulinic acid inhibited P-Selectin binding to 2,3, sLex, a chemical known to bind to P-Selectin, with an IC₅₀ of 125 uM. Also it inhibited P-Selectin binding to HL-60 cells in a dose-dependent way with an IC₅₀ of 0.75 mM. Betulinic acid and derivatives also significantly interfere with the binding to colon cancer cells, LS174T to E-Selectin.

Dasgupta et al (WO 96/29068) disclosed a method and composition for inhibiting tumor growth using the active compound betulinic acid. The invention provides a method and composition for inhibiting tumor growth and, particularly, for inhibiting growth of melanoma using a natural product derived compound. The invention also provides a treatment method using betulinic acid to prevent growth or spread of cancer cells, wherein betulinic acid is applied in a topical preparation.

Pezzuto et al (US Patent 5,869,535) disclose method and composition for probes inhibiting tumor growth using betulinic acid or a derivative thereof. Betulinic acid has been isolated from stem bark of *Ziziphus mauritiana*, by mediating a selective cytotoxic profile against human melanoma in a subject panel of human cancer cell lines, conducting a bioassay directed fractionation based on the profile of bioactivity using cultured human melanoma cells (MEL-2) as the monitor, and betulinic acid has been obtained therefrom as the active compound. The resulting betulinic acid can be used to inhibit tumor growth, or can be converted to a derivative to prevent which prevents or inhibits tumor growth. The invention also provides a treatment method using betulinic acid to prevent the growth or spread of cancerous cells, wherein betulinic acid or derivatives thereof is applied in a topical preparation. Betulinic acid was found to inhibit in vitro growth of MEL-2 cells. However, none of the other cell lines tested [A431 (squamous cells), BC-1 (breast), COL-2 (colon), HT1080 (sarcoma), KB (human oral epidermoid carcinoma), LNCaP (prostate), LU-1 (lung), U373 (glioma) and ZR-75-1 (breast)] were affected by betulinic acid (ie., ED₅₀ values of greater than 20 µg/ml).

Lee et al (WO 96/39033) disclose betulinic acid and dihydrobetulinic acid acyl derivative to have potent anti-HIV activity. The C₃-hydroxy, C₁₇-carboxylic acid and C₂₀-exomethylene groups have been modified. Anti-HIV assays indicate potent anti-HIV activity of betulinic acid and dihydrobetulinic acid derivatives in acutely infected H9 lymphocytes with EC₅₀ values of less than 1.7 x 10⁻⁵ µM respectively.

WO 98/51294 and WO 98/51293 disclose betulinic acid compounds to be used in the treatment of cancer.

### Objects of the invention

This invention provides for novel betulinic acid derivatives and compositions containing them with pharmaceutically acceptable additives, diluents, carriers and excipients with or without betulinic acid.

Another object of the invention relates to providing novel betulinic acid derivatives, which are used for inhibiting angiogenesis.

Another object of the invention is to provide a compound and compositions for treating, inhibiting and/or preventing angiogenesis using a natural product-derived compound and its derivatives.

Furthermore the application discloses the use of derivatives according to the formula of fig. 1 to manufacture a composition useful in the treatment of a patient with angiogenesis, wherein the derivatives are administered systemically.

Yet another object of the invention is to overcome the problem of high toxicity associated with standard antiangiogenic chemotherapeutic agents by using a natural product-derived compound, e.g., betulinic acid or its derivatives.

Still another object of the invention is to overcome the problem of insufficient availability associated with synthetic antiangiogenic anticancer agents by using readily available semisynthetic derivatives of betulinic acid.

Another object of the invention is to overcome the problem of high costs of synthetic antiangiogenic agents by utilizing the readily available natural product derived compound. e.g. betulinic acid and its derivatives which is expected to be less expensive than other chemotherapeutic drugs.

### Summary of the invention

The above objects and others have been achieved by providing novel betulinic acid derivatives of formulae of figure 1 which are described in the present description.

### Detailed Description of Invention

The present invention provides a pharmaceutical composition useful for preventing / inhibiting angiogenesis. Betulinic acid derivatives inhibit endothelial cell proliferation and exhibiting high endothelial cell specificity thereby specifically targeting endothelial cells. The derivatives also inhibit the formation of tube-like-structures (TLS) of endothelial cells when grown on Matrigel coated surface. The endothelial cell anti-proliferative activity along with anti-TLS activity very strongly suggests the anti-angiogenic activity of betulinic acid derivatives.

The novel derivatives of betulinic acid have a basic skeleton of betulinic acid as shown herebelow in Figure 1.

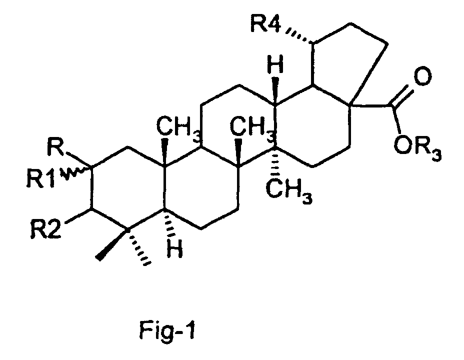

Wherein R, R1, R2, R3 and R4 independently or in combination represent the following groups:-
- R =: H
- R1 =: H,Br
- R2 =: O, OH,NNHC6H5, NHNHC6H5, NHNHC6H4(OCH3) [4], OCOC6H3F2[3,5], OCOC6H3F2[2,4], OCOC6H4(CF3)[3],OCOC6H4(CF3)[2], N=CHC6H3F2[2,4],
N=CHC6H3F2[3,4], NOCH2C6H4NO2[4], OCOC6H4(C5H11)[4], OCOC6H4(C7H15)[4], OCOC6H4(OCH3)2[2,5], OCOCH2C6H3(OCH3)2[3,4], OCOCH2C6H3(OCH3)2[2,4], OCOC6F5, NOH, OCOCClF2, OCOC6H4-C6H5, OCOCH(Cl)C6H5, OCO-(CH2)3COOH, OCOC6H4Cl, OCOC6H4(CHCL2)(3), OSO2CH2CH2CH2Cl, OCOC6H2(COOH)(2)Cl2[3,6], OCOCH(Cl)-CH3, NNHCOC6H4(OH)[2],
- R3 =: H, CH2CH2COOCH3, COCH=CH2, 3-deoxy dihydro betulinic acid
- R4: =CH2=CCH3, BrCH2-C(Br)CH3, CH(CH3)2.

### Preparation of Betulinic acid derivatives.

The following procedures are either used alone or in combination to produce the derivatives of the present invention.

### Example 1

### Preparation of 3-o-acyl derivatives

Method 1: Substrate in organic base is treated with suitable anhydride at room temperature for approximately 4-16 hours. Examples of anhydrides that can be used in this process are represented by general formula (RCH₂CO₂)O wherein R=H, CH₃, C₂H₅, etc. The reaction was worked by evaporation of the reaction mixture, addition of water and extraction with an organic solvent. The organic layer was dried over anhydrous sodium sulfate, evaporated and residue crystallized to yield the corresponding pure 3-0 acyl derivatives respectively. Examples of organic bases that can be used in this method are TEA, pyrdine and DMPA.

Method II: Substrate in halogenated organic solvent was treated with suitable acyl chloride as in Method 1. The reaction was worked up as described in Method I to yield the corresponding 3-o-acy' derivatives in the pure form. Examples of acyl chlorides that can be used are RCH₂(CH₂)ₙ, COCI wherein R=H, Cl or F Br and n=0 to 9 or RCH₂(CH)ₙXCOCI wherein R=H, X=OH, OCOCH₃ and n=1. The halogenated solvent may be selected from CCl₄ CH₂ Cl₂, C₆ H₅CH₃ or the like.

### Example 2

### Preparation of 3-oxo-derivatives.

The substrate was dissolved in an organic solvent and conventional oxidizing agent was added under normal reaction conditions. The reaction was worked up as described in Method I to yield the corresponding 3-oxo derivatives in the pure form.

Example of oxidizing agents that can be used are CrO₃/Py; CrO₃/H₂SO₄; CrO₃./AcOH or the like. The normal reaction condition is stirring the substrate with oxidizing agent at from 0°C to room temperature for a few hours. The orgainc solvent may be selected from acetone, CH₂Cl₂, AcOH, mixtures thereof or the like.

### Example 3

### Preparation of 2, 20,29-tribromo 3-oxo derivative

A -3-oxo betulinic acid derivative prepared according to the process of Example 3 was dissolved in halogenated organic solvent. To this was added dropwise liquid bromine dissolved in the same solvent and the temperature was maintained between 0-10°C. The reaction mixture was brought to room temperature and stirred for a few hours. The reaction was worked up as described in Method I of Example 2. The organic layer was washed with 5-10% aqueous alkaline solution and evaporated. The crystallized product yielded pure 2, 20, 29-tribromo-3-oxo derivatives. Examples of halogenated solvents that can be used are CCl₄,CH₂Cl₂, CHCl₃ and the like; Examples of aqueous alkaline solution that can be used are bicarbonate or carbonate of an alkali metal in water, and the like.

3-Oxo-derivative of betulinic acid, dihydrobetulinic acid or their derivatives can be used in the processes of Examples 4, 5, 8,10 and 14.

### Example 4

### Preparation of 3-oximino derivative

A 3-oxo derivative is mixed in an alcoholic solvent such a methanol, ethanol, propanol and the like. To this was added hydroxylamine, phenyl hydroxylamine or benzyl hydroxylamine or its substituted derivatives and sodium acetate. The mixture was refluxed for a few hours. The reaction mixture was evaporated to dryness. The reaction was worked up as described in Method I of Example 2 and yielded crude-3-oximino derivative which crystallized to yield the corresponding pure 3-oximino derivative.

### Example 5

### Preparation of phenylhydrazone of 3-oxo derivative

Phenylhydrazine or its phenyl substituted analogs or a salt thereof, and sodium acetate were added to 3-oxo derivative dissolved in alcoholic solvent such as methanol, ethanol, propanol and the like, and was refluxed for about four hours. The reaction was worked up as described in Method I of Example 2 to yield the corresponding pure phenylhydrazone derivative in pure form.

### Example 6

### Preparation of 17 and /or 20-carboxyalkyl carboxylate

To the substrate dissolved in dry dimenthylformamide, sodium hydride was added and the mixture was stirred at room temperature for about two hours. A suitable haloalkyl carboxyester was added to the above reaction mixtures and the mixture was stirred at room temperature for 16-20 hours. The reaction was worked up as described in Method I of Example 2 to yield pure 17 and/or 20-carboxyalky carboxylate derivative. Examples of haloalkyl carboxy esters that can be used are chloro or bromo derivative of methyl or ethyl acetate, or chloro or bromo derivative of propionate and the like.

### Example 10

### Preparation of 3-amino derivatives

a] 3-oximino derivative is dissolved in glacial acetic acid and shaken under hydrogen atmosphere (60-70psi) in presence of platinum oxide catalyst for several hours. Reaction mixture is filtered, mother liquor evaporated under vacuum to remove glacial acetic acid and the residue worked up in the usual manner to yield the corresponding 3-amino derivative.
b] 3 oxo-derivative is dissolved in methanol added ammonium sulphate and sodium borodhyride and reflected for 2-4 hrs. Reaction mixture evaporated to dryness, added water, filtered the solid and crystallized to give 3-amino derivatives.

### Example 11

### Preparation of 3-o- benzoyl derivatives

Substrate in organic base is treated with suitable benzoyl chloride for approximately 6-16 hours at an ambient temperature. Examples of benzoyl chloride that can be used are represented by general formula Rₙ(Ar)CoCl wherein n = 1 to 3, R = H, Cl, Br, F, CF₃ and Ar = C₆H₅, C₆H₄, C₆H₃ or C₆H₂. The reaction was worked up by addition of water and extraction with organic solvent. The organic layer was dried over anhydrous sodium sulphate, evaporated and residue crystallized to yield pure 3-o-benzoyl derivatives respectively. Examples of organic bases that can be used are pyridine, piperidine.

### Example 13

### Preparation of 3-phenyl hydrazino or its phenyl substituted derivative

3-phenylhydrazone or its phenyl substituted derivative of betulinic acid or dihydrobetulinic acid is dissolved in glacial acetic acid and shaken under hydrogen atmosphere (50-70- psi) in presence of platinum sponge catalyst for 3-5 hours. Reaction mixture was filtered, mother liquor evaporated under vacuum to remove glacial acetic acid and the residue crystallized from alcoholic solvent to yield pure 3-phenyl hydrazino or its phenyl substituted derivative. Alcoholic solvents used are methanol, ethanol or iso propanol.

### Example 14

### Preparation of 3-N-Hydroxyethyl derivative

3-oxo-derivative is dissolved in absolute alcoholic solvent such as methanol / ethanol and to it added 15-20% alcoholic hydrochloric acid and 2-aminoethanol and stirred at room temperature for 30 - 60 minutes. To this added sodium cyanoborohydride and further stirred at room temperature for approximately 72 hours. Worked up by adding water followed by filtration of solid to yield crude product, which was crystallized from alcohol to yield pure 3-N-hydroxyethyl derivative.

### Example 15

### Preparation of 3-N-Benzylidene derivative

3-amino derivative is dissolved in alcoholic solvent, such as methanol / ethanol and to it added benzaldehyde or substituted benzaldehyde derivative in presence or absence of alkali carbonate, such as sodium or potassium carbonate. The mixture was stirred for few hours at ambient temperature to 50°C approximately. The reaction mixture was worked up by removing alcohol under vacuum and addition of water. The aqueous layer either filtered or extracted with halogenated organic solvent, followed by evaporation yielded 3-N-benzylidene derivative.

### Example 16

30µl of ECV304 cell suspension (50 x 10⁴ cells/ml in RPMI 1640 containing 10% FBS) followed by 150µl of medium was added to the wells of a 96-well tissue culture plate (Nunc, Denmark) and incubated (37°C, 5% CO₂) overnight. 20µl of the betulinic acid derivative to be tested was then added at concentrations ranging from 0.5ug/ml to 4 ug/ml. Each concentration was plated in triplicates. 20µl of medium alone was added to control wells. After 72 hours of incubation an MTT assay (Mosmann, 1983) was performed and percentage inhibition in proliferation of treated cells was calculated with respect to control cells.

The cytotoxicity assays for tumor cells have been described in detail in our application No. 09/040,856 filed in US on March 18, 1998. Table I shows the ED₅₀ values against ECV304 and four different tumor cell lines and the endothelial cell specificities of seventeen potent derivatives.

**Table - I**

| **S.No** | **Derivative** | **Endothelial** | **Prostate** | | **Lung** | | **Ovary** | | **Colon** | |
|---|---|---|---|---|---|---|---|---|---|---|
| | | **ECV304** **(ED**_{**50**}**)** | **DU145** **(ED**_{**50**}**)** | **ECS** **Ratio** | **L132** **(ED**_{**50**}**)** | **ECS** **Ratio** | **PA-1** **(ED**_{**50**}**)** | **ECS** **Ratio** | **HT-29** **(ED**_{**50**}**)** | **ECS** **Ratio** |
| **1** | **MJ353-RS** | 2.4 | 6.5 | 2.7 | 4.5 | 1.9 | 5 | 2.08 | > 10 | 4.2 |
| **2** | **MJ548-RS** | 2.5 | > 10 | > 4 | > 10 | > 4 | 3.9 | 1.56 | > 10 | 4.0 |
| **3** | **MJ878-RS** | 0.5 | 9.9 | 19.8 | 0.8 | 1.6 | 3.5 | 7.0 | 1.75 | 3.5 |
| **4** | **MJ912-RS** | 0.4 | 8.5 | 21.1 | > 4 | >10 | ND | - | 0.35 | 0.87 |
| **5** | **MJ935-RS** | 0.7 | 3.2 | 4.5 | 1.2 | 1.7 | ND | - | > 10 | > 14.3 |
| **6** | **MJ937-RS** | 0.7 | 2.5 | 3.5 | 1.1 | 1.5 | 1.6 | 2.3 | 1.7 | 2.4 |
| **7** | **MJ939-RS** | 0.9 | > 10 | > 11.1 | 2.7 | 3.0 | > 4 | > 4.4 | > 10 | > 11.1 |
| **8** | **MJ943-RS** | 2.6 | > 4 | > 1.5 | 4 | 1.5 | > 4 | > 1.53 | > 10 | 3.84 |
| **9** | **MJ998-RS** | 0.35 | 2.2 | 6.2 | 2.5 | 7.1 | 1.3 | 3.7 | 4 | 11.4 |
| **10** | **MJ1065-RS** | 2.4 | 2.5 | 1.04 | 3.4 | 1.4 | 1.3 | 0.54 | 4.9 | 2.04 |
| **11** | **MJ1098-RS** | 0.6 | 1.5 | 2.5 | 1.3 | 2.1 | 1.6 | 2.7 | 2.6 | 4.3 |
| **12** | **MJ1101-RS** | 4.0 | > 10 | > 2.5 | 1.7 | 0.43 | > 4 | > 1 | > 10 | 2.5 |
| **13** | **MJ1103-RS** | 2.0 | > 4 | > 2.0 | 4 | 2.0 | 1.5 | 0.75 | 10 | 5.0 |
| **14** | **MJ1104-RS** | 1.9 | 2 | 1.05 | 5.9 | 3.1 | > 4 | > 2.1 | 3.5 | 1.84 |
| **15** | **MJ1108-RS** | 1.8 | 1 | 0.55 | 4.6 | 2.5 | ND | - | > 10 | > 5.6 |
| **16** | **MJ1138-RS** | 1.7 | > 10 | > 5.8 | 7 | 4.1 | > 4 | >2.3 | > 10 | > 5.89 |
| **17** | **MJ1161-RS** | 4.0 | 0.4 | 0.1 | 3.5 | 0.88 | 2.6 | 0.65 | 3.5 | 0.87 |
| ED₅₀ = Concentration (µg/ml) of drug that causes 50%Cytotoxicity, where Percent Cytotoxicity = [ 1 - O.D. _{Treated} / O.D. _{Control}] * 100 ECS ratio = ED₅₀ Tumor cell growth / ED₅₀ Endothelial cell growth. ECS less than 10 = Low ECS ECS between 10 and 20 = Moderate ECS ECS greater than 20 = High ECS | | | | | | | | | | |

We predict that the 'high' and 'moderate' ECS compounds specifically target endothelial cells and can be grouped under potent anti-angiogenic compounds while 'low' ECS compounds would supplement their already reported cytotoxic activity against tumor cells.

### Example 17

Several derivatives of betulinic acid were prepared by making substitutions and/or structural changes at C_{3,} C_{17,} C₂₀, and/or C₂₉ positions of betulinic acid as described in the examples. The derivatives were characterized on the basis of spectral data. Table II refers to the structures of Figure 2 wherein R to R₄, which are clearly indicated, list the structures of forty derivatives. Figure 2 wherein R to R₄ are shown herebelow:

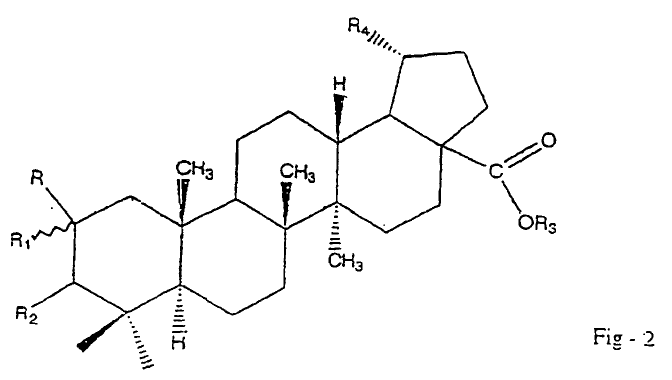

**TABLE II**

| **DERIVATIVE** | **R** | **R1** | **R2** | **R3** | **R4** |
|---|---|---|---|---|---|
| MJ353-RS | H | H | =NNHC₆H₅ | H | CH₂=CCH₃ |
| MJ548-RS | H | Br | =O | CH₂CH₂COO-CH₃ | BrCH₂-C(Br)CH₃ |
| MJ-878-RS | H | H | -NHNH C₆H₅ | H | -CH(CH₃)₂ |
| MJ-912-RS | H | H | -NHNH C₆H₄ (OCH₃)[4] | H | -CH(CH₃)₂ |
| MJ-935-RS | H | H | -OCO-C₆H₃F₂[3,5] | H | -CH(CH₃)₂ |
| MJ-937-RS | H | H | -OCO-C₆H₃F₂[2,4] | H | -CH(CH₃)₂ |
| MJ939-RS | H | H | -OCOC₆H₄(CF₃)[3] | H | CH₂=C-CH₃ |
| MJ943-RS | H | H | -OCOC₆H₄(CF₃)[2] | H | -CH(CH₃)₂ |
| MJ998-RS | H | H | -N=CHC₆H₃F₂[3,4] | H | -CH(CH₃)₂ |
| MJ1065-RS | H | H | -N=CHC₆H₃F₂(2,4) | H | -CH(CH₃)₂ |
| MJ1098-RS | H | H | NOCH₂C₆H₄NO₂(4) | H | -CH(CH₃)₂ |
| MJ1101-RS | H | H | -OH | COCH₂=CH₂ | CH₂=C-CH₃ |
| MJ1103-RS | H | H | -OH | COCH₂=CH₂ | -CH(CH₃)₂ |
| MJ1104-RS | H | H | -OCOC₆H₄(C₅H₁₁)[4] | H | CH₂=C-CH₃ |
| MJ1108-RS | H | H | -OCOCH₂C₆H₃(OCH₃)₂[2,5] | H | CH₂=C-CH₃ |
| MJ1138-RS | H | H | -OCOC₆H₄(C₇H₁₅)[4] | H | CH₂=C-CH₃ |
| MJ1161-RS | H | H | -OH | 3-deoxy DHBA* | -CH(CH₃)₂ |
| MJ1183-RS | H | H | -OCOCH₂C₆H₃(OCH₃)₂[3,4] | H | CH₂=C-CH₃ |
| MJ1204-RS | H | H | =O | COCH=CH₂ | -CH(CH₃)₂ |
| MJ1205-RS | H | H | -OCO C₆F₅ | H | CH₂=C-CH₃ |
| MJ1210-RS | H | H | =NOH | COCH=CH₂ | -CH(CH₃)₂ |
| MJ1213-RS | H | H | -OCO C₆F₅ | H | -CH(CH₃)₂ |
| MJ1283-RS | H | H | -OCOC₆H₃(OCH₃)₂[3,4] | H | -CH(CH₃)₂ |
| MJ1287-RS | H | H | -OCOC₆H₃(OCH₃)₂[2,4] | H | -CH(CH₃)₂ |
| MJ1295-RS | H | H | -OCOCClF₂ | H | -CH(CH₃)₂ |
| MJ1296-RS | H | H | -OCOC₆H₅-C₆H₅ | H | CH₂=C-CH₃ |
| MJ1298-RS | H | H | -OCOCH(Cl)Ph | H | CH₂=C-CH₃ |
| MJ1301-RS | H | H | -OCO-(CH₂)₃COOH | H | -CH(CH₃)₂ |
| MJ1304-RS | H | H | -OCOC₆H₄Cl(4) | H | CH₂=C-CH₃ |
| MJ1305-RS | H | H | -OCOC₆H₄Cl(4) | H | -CH(CH₃)₂ |
| MJ1315-RS | H | H | -OCOC₆H₄(CHCl₂)(3) | H | CH₂=C-CH₃ |
| MJ1316-RS | H | H | -OCOC₆H₄(CHCl₂)(3) | H | -CH(CH₃)₂ |
| MJ1312-RS | H | H | -OSO₂CH₂CH₂CH₂Cl | H | CH₂=C-CH₃ |
| MJ1313-RS | H | H | -OSO₂CH₂CH₂CH₂Cl | H | -CH(CH₃)₂ |
| MJ1327-RS | H | H | -OCOC₆H₂(COOH)(2)Cl₂(3,6) | H | CH₂=C-CH₃ |
| MJ1328-RS | H | H | -OCOC₆H₂(COOH)(2)Cl₂(3,6) | H | -CH(CH₃)₂ |
| MJ1335-RS | H | H | -OCOCH(Cl)-CH₃ | H | CH₂=C-CH₃ |
| MJ1336-RS | H | H | -OCOCH(Cl)-CH₃ | H | -CH(CH₃)₂ |
| MJ1338-RS | H | H | =NNHCOC₆H₄(OH)(2) | H | -CH(CH₃)₂ |
| MJ1366-RS | H | Br | =N-O-CH₂C₆H₄NO₂(4) | H | -CH(CH₃)₂ |

| | | | | | |
|---|---|---|---|---|---|
| * *Dihydrobetulinic acid* | | | | | |

### Example 18

Matrigel (70 µl) was placed into each well of a 96-well culture plate at 4°C and was allowed to polymerize by incubation at 37°C for 30 min. ECV304 (1 x 10⁴) cells were seeded on the Matrigel in 200 µl DMEM containing 10% FBS. Betulinic acid and derivatives to be tested were added to marked wells at non-cytotoxic concentrations and incubated at 37°C for 24 - 48 hours. The absence of cytotoxicity of betulinic acid and its derivatives on ECV304 cells at the above time points was confirmed by suitable controls. Five different phase-contrast microscopic fields (4X) were viewed and total tube area of the tube-like-structures (TLS) measured using Video Pro 32 Image Analysis system. Percent reduction in total tube area was given as the mean of the data from five fields. Percent inhibition of TLS was calculated with reference to Controls(no drug).

**Table - III**

| **Derivative** | **% reduction in total tube area at concentration** | | |
|---|---|---|---|
| | 0.5 µg/ml | 2 µg/ml | 4 µg/ml |
| **MJ937-RS** | 16.7 | 21 | 21.7 |
| **MJ998-RS** | 21.1 | 13.4 | 33.4 |
| **MJ1065-RS** | 23.4 | 16.7 | 46.7 |
| **MJ1098-RS** | 15 | 7.5 | 10 |
| **MJ1161-RS** | 18.4 | 16.7 | 11.7 |

### Example 19

A suitable formulation of betulinic acid derivatives was prepared as follows. The derivatives were solubilized in a minimum volume of methanol. The derivatives may also be solubilized in isopropyl alcohol, dimethylformamide, dimethylsulfoxide or any other suitable solvent. Substituted beta-cyclodextrin, such as 2-hydroxypropyl beta-cyclodextrin, sulfobutyl ether beta-cyclodextrin was separately dissolved in water to a concentration of approximately 50 to 1000 mg per ml, preferably 250 to 750 mg per ml. The solubilized betulinic acid derivative was added in small aliquots to the derivatized beta cyclodextrin solution and sonicated at low temperature until a clear solution developed. The organic solvent was then removed by rotary evaporation and the final solution filtered to give a sterile product. The resulting solution was lyophilized.

Systemic administration refers to oral, rectal, nasal, transdermal and parentral (i.e., intramuscular, intraperitoneal, subcutaneous or intravenous). In accordance with good clinical practice, it is preferred to administer the composition at a dose that will produce antiangiogenic effects without causing undue harmful side effects. The composition may be administered either alone or as a mixture with other therapeutic agents.

Pharmaceutical compositions which provide from about 10 mg to 1000 mg of the composition per unit dose are preferred as tablets, lozenges, capsules, powders, aqueous or oily suspensions, syrups, elixirs, implants or aqueous solutions by any conventional method. The nature of pharmaceutical composition employed will, of course, depend on the desired route of administration. The human dosage of the composition is in the range of 1.0 to 200 mg/kg/day and the preferred range is 1.0 to 50 mg/kg/day.

One embodiment of the invention relates to a method of using novel betulinic acid derivatives or a combination thereof for the manufacture of a medicament to treat a patient with tumor associated angiogenesis by administering a pharmaceutically effective dosage of said betulinic acid derivative or its combination to the patient. The patient of the invention can be human, mammal or other animal. The ED₅₀ value of betulinic acid derivatives against human umbilical vein endothelial cells is 0.35 to 4.0 µg/ml. The endothelial cell specificity of betulinic acid derivatives for prostate cancer is 1.04 to 21.1. As regards the endothelial cell specificity of betulinic acid derivatives for lung cancer is 0.43 to >10. However, regarding the endothelial cell specificity of betulinic acid derivatives for ovarian cancer is 0.54 to 7.0. With regard to the endothelial cell specificity of betulinic acid derivatives for colon cancer is 0.87 to 14.3.

The betulinic acid derivative is administered to the patient in a pharmaceutically acceptable additive, carrier, diluent, solvent, filler, lubricant, excipient, binder or stabilizer. Preferably, the betulinic acid derivative is administered in the form of a tablet, lozenge, capsule, powder, aqueous or oily suspension, syrup, elixir, implant or aqueous solution and the betulinic acid derivative or derivatives or combination thereof is administered to the patient systemically.

## Claims

1. A betulinic acid derivative, represented by the following formula: 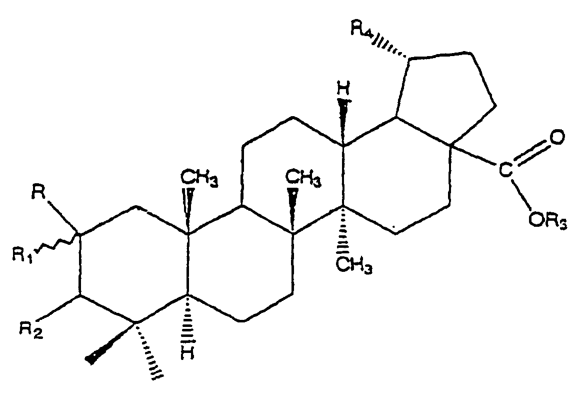 wherein a combination of R, R₁, R₂, R₃ and R₄ is selected from the group comprising
| DERIVATIVE | R | R1 | R2 | R3 | R4 |
|---|---|---|---|---|---|
| MJ353-RS | H | H | =NNHC₆H₅ | H | CH₂=CCH₃ |
| MJ548-RS | H | Br | =O | CH₂CH₂COO-CH₃ | BrCH₂-C(Br)CH₃ |
| MJ-878-RS | H | H | -NHNH C₆H₅ | H | -CH(CH₃)₂ |
| MJ-912-RS | H | H | -NHNH C₆H₄(OCH₃)[4] | H | -CH(CH₃)₂ |
| MJ-935-RS | H | H | -OCO-C₆H₃F₂[3,5] | H | -CH(CH₃)₂ |
| MJ-937-RS | H | H | -OCO-C₆H₃F₂[2,4] | H | -CH(CH₃)₂ |
| MJ939-RS | H | H | -OCOC₆H₄(CF₃)[3] | H | CH₂=C-CH₃ |
| MJ943-RS | H | H | -OCOC₆H₄(CF₃)[2] | H | -CH(CH₃)₂ |
| MJ998-RS | H | H | -N=CHC₆H₃F₂[3,4] | H | -CH(CH₃)₂ |
| MJ1065-RS | H | H | -N=CHC₆H₃F₂(2,4) | H | -CH(CH₃)₂ |
| MJ1098-RS | H | | H NOCH₂C₆H₄NO₂(4) | H | -CH(CH₃)₂ |
| MJ1101-RS | H | H | -OH | COCH₂=CH₂ | CH₂=C-CH₃ |
| MJ1103-RS | H | H | -OH | COCH₂=CH₂ | -CH(CH₃)₂ |
| MJ1104-RS | H | H | -OCOC₆H₄(C₅H₁₁)[4] | H | CH₂=C-CH₃ |
| MJ1108-RS | H | H | -OCOCH₂C₆H₃(OCH₃)₂[2,5] | H | CH₂=C-CH₃ |
| MJ1138-RS | H | H | -OCOC₆H₄(C₇H₁₅)[4] | H | CH₂=C-CH₃ |
| MJ1161-RS | H | H | -OH | 3-deoxy DHBA* | -CH(CH₃)₂ |
| MJ1183-RS | H | H | -OCOCH₂C₆H₃(OCH₃)₂[3,4] | H | CH₂=C-CH₃ |
| MJ1204-RS | H | H | =O | COCH=CH₂ | -CH(CH₃)₂ |
| MJ1205-RS | H | H | -OCO C₆F₅ | H | CH₂=C-CH₃. |
| MJ1210-RS | H | H | =NOH | COCH=CH₂ | -CH(CH₃)₂ |
| MJ1213-RS | H | H | -OCO C₆F₅ | H | -CH(CH₃)₂ |
| MJ1283-RS | H | H | -OCOC₆H₃(OCH₃)₂[3,4] | H | -CH(CH₃)₂ |
| MJ1287-RS | H | H | -OCOC₆H₃(OCH₃)₂[2,4] | H | -CH(CH₃)₂ |
| MJ1295-RS | H | H | -OCOCClF₂ | H | -CH(CH₃)₂ |
| MJ1296-RS | H | H | -OCOC₆H₄C₆H₅ | H | CH₂=C-CH₃ |
| MJ1298-RS | H | H | -OCOCH(Cl)Ph | H | CH₂=C-CH₃ |
| MJ1301-RS | H | H | -OCO-(CH₂)₃COOH | H | -CH(CH₃)₂ |
| MJ1304-RS | H | | H -OCOC₆H₄Cl(4) | H | CH₂=C-CH₃ |
| MJ1305-RS | H | H | -OCOC₆H₄Cl(4) | H | -CH(CH₃)₂ |
| MJ1315-RS | H | H | -OCOC₆H₄(CHCl₂(3) | H | CH₂=C-CH₃ |
| MJ1316-RS | H | H | -OCOC₆H₄(CHCl₂)(3) | H | -CH(CH₃)₂ |
| MJ1312-RS | H | H | -OSO₂CH₂CH₂CH₂Cl | H | CH₂=C-CH₃ |
| MJ1313-RS | H | H | -OSO₂CH₂CH₂CH₂Cl | H | -CH(CH₃)₂ |
| MJ1327-RS | H | H | -OCOC₆H₂(COOH)(2)Cl₂(3,6) | H | CH₂=C-CH₃ |
| MJ1328-RS | H | H | -OCOC₆H₂(COOH)(2)Cl₂(3,6) | H | -CH(CH₃)₂ |
| MJ1335-RS | H | H | -OCOCH(Cl)-CH₃ | H | CH₂=C-CH₃ |
| MJ1336-RS | H | H | -OCOCH(Cl)-CH₃ | H | -CH(CH₃)₂ |
| MJ133S-RS | H | H | =NNHCOC₆H₄(OH)(2) | H | -CH(CH₃)₂ |
| MJ1366-RS | H | Br | =N-O-CH₂C₆H₄NO₂(4) | H | -CH(CH₃)₂ |
| | | | | | |
|---|---|---|---|---|---|
| **Dihydrobetulinic acid* | | | | | |

2. Use of a betulinic acid derivative or a combination of several betulinic acid derivatives according to claim 1 to manufacture a composition useful in the treatment of a patient with angiogenesis.

3. Use according to claim 2, wherein the angiogenesis is tumor associated.

4. Use as claimed in claim 3 wherein said patient is human, mammal or other animal.

5. Use as claimed in claim 3 wherein ED₅₀ value of betulinic acid derivatives against human umbilical vein endothelial cells is 0.35 to 4.0 µg/ml.

6. Use as claimed in claim 3 wherein the endothelial cell specificity of betulinic acid derivatives for prostate cancer is 1.04 to 21.1.

7. Use as claimed in claim 3 wherein the endothelial cell specificity of betulinic acid derivatives for lung cancer is 0.43 to > 10.

8. Use as claimed in claim 3 wherein the endothelial cell specificity of betulinic acid derivatives for ovarian cancer is 0.54 to 7.0.

9. Use as claimed in claim 3 wherein the endothelial cell specificity of betulinic acid derivatives for colon cancer is 0.87 to 14.3.

10. Use as claimed in claim 3 wherein the betulinic acid derivative is administered to the patient in a pharmaceutically acceptable additive, carrier, diluent, solvent, filler, lubricant, excipient, binder or stabilizer.

11. Use as claimed in claim 3 wherein the betulinic acid derivative is administered in the form of a tablet, lozenge, capsule, powder, aqueous or oily suspension, syrup, elixir, implant or aqueous solution.

12. Use as claimed in claim 3 wherein the dosage for human is in the range of 1.0 to 200 mg/kg/day.

13. Use as claimed in claim 3 wherein the betulinic acid derivative or derivatives or combination thereof is administered to the patient systemically.

## Patentansprüche

1. Ein Betulinsäurederivat, dargestellt durch die folgende Formel: 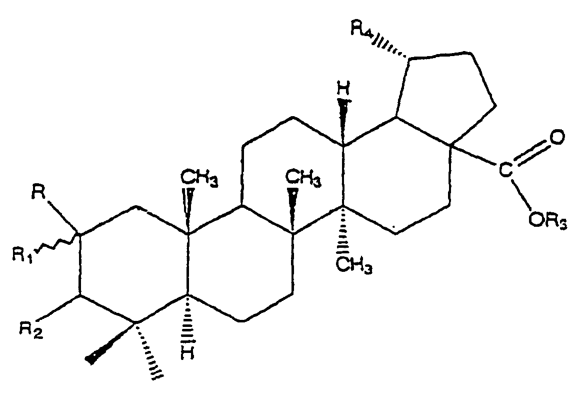 wobei eine Kombination aus R, R₁, R₂, R₃ und R₄ ausgewählt ist aus der Gruppe, umfassend
| DERIVAT | R | R1 | R2 | R3 | R4 |
|---|---|---|---|---|---|
| MJ353-RS | H | H | =NNHC₆H₅ | H | CH₂=CCH₃ |
| MJ548-RS | H | Br | =O | CH₂CH₂COO-CH₃ | BrCH₂-C(Br)CH₃ |
| MJ-878-RS | H | H | -NHNH C₆H₅ | H | -CH(CH₃)₂ |
| MJ-912-RS | H | H | -NHNH C₆H₄(OCH₃)[4] | H | -CH(CH₃)₂ |
| MJ-935-RS | H | H | -OCO-C₆H₃F₂[3,5] | H | -CH(CH₃)₂ |
| MJ-937-RS | H | H | -OCO-C₆H₃F₂[2,4] | H | -CH(CH₃)₂ |
| MJ939-RS | H | H | -OCOC₆H₄(CF₃)[3] | H | CH₂=C-CH₃ |
| MJ943-RS | H | H | -OCOC₆H₄(CF₃)[2] | H | -CH(CH₃)₂ |
| MJ998-RS | H | H | -N=CHC₆H₃F₂[3,4] | H | -CH(CH₃)₂ |
| MJ1065-RS | H | H | -N=CHC₆H₃F₂(2,4) | H | -CH(CH₃)₂ |
| MJ1098-RS | H | H | NOCH₂C₆H₄NO₂(4) | H | -CH(CH₃)₂ |
| MJ1101-RS | H | H | -OH | COCH₂=CH₂ | CH₂=C-CH₃ |
| MJ1103-RS | H | H | -OH | COCH₃=CH₂ | -CH(CH₃)₂ |
| MJ1104-RS | H | H | -OCOC₆H₄(C₅H₁₁)[4] | H | CH₂=C-CH₃ |
| MJ1108-RS | H | H | -OCOCH₂C₆H₃(OCH₃)₂[2,5] | H | CH₂=C-CH₃ |
| MJ1138-RS | H | H | -OCOC₆H₄(C₇H₁₅)[4] | H | CH₂=C-CH₃ |
| MJ1161-RS | H | H | -OH | 3-deoxy DHBA* | -CH(CH₃)₂ |
| MJ1183-RS | H | H | -OCOCH₂C₆H₃(OCH₃)₂[3,4] | H | CH₂=C-CH₃ |
| MJ1204-RS | H | H | =O | COCH=CH₂ | -CH(CH₃)₂ |
| MJ1205-RS | H | H | -OCO C₆F₅ | H | CH₂=C-CH₃ |
| MJ1210-RS | H | H | =NOH | COCH=CH₂ | -CH(CH₃)₂ |
| MJ1213-RS | H | H | -OCO C₆F₅ | H | -CH(CH₃)₂ |
| MJ1283-RS | H | H | -OCOC₆H₃(OCH₃)₂[3,4] | H | -CH(CH₃)₂ |
| MJ1287-RS | H | H | -OCOC₆H₃(OCH₃)₂[2,4] | H | -CH(CH₃)₂ |
| MJ1295-RS | H | H | -OCOCClF₂ | H | -CH(CH₃)₂ |
| MJ1296-RS | H | H | -OCOC₆H₅-C₆H₅ | H | CH₂=C-CH₃ |
| MJ1298-RS | H | H | -OCOCH(Cl)Ph | H | CH₂=C-CH₃ |
| MJ1301-RS | H | H | -OCO-(CH₂)₃COOH | H | -CH(CH₃)₂ |
| MJ1304-RS | H | H | -OCOC₆H₄Cl(4) | H | CH₂=C-CH₃ |
| MJ1305-RS | H | H | -OCOC₆H₄Cl(4) | H | -CH(CH₃)₂ |
| MJ1315-RS | H | H | -OCOC₆H₄(CHCl₂)(3) | H | CH₂=C-CH₃ |
| MJ1316-RS | H | H | -OCOC₆H₄(CHCl₂)(3) | H | -CH(CH₃)₂ |
| MJ1312-RS | H | H | -OSO₂CH₂CH₂CH₂Cl | H | CH₂=C-CH₃ |
| MJ1313-RS | H | H | -OSO₂CH₂CH₂CH₂Cl | H | -CH(CH₃)₂ |
| MJ1327-RS | H | H | -OCOC₆H₂(COOH)(2)Cl₂(3,6) | H | CH₂=C-CH₃ |
| MJ1328-RS | H | H | -OCOC₆H₂(COOH)(2)Cl₂(3,6) | H | -CH(CH₃)₂ |
| MJ1335-RS | H | H | -OCOCH(Cl)-CH₃ | H | CH₂=C-CH₃ |
| MJ1336-RS | H | H | -OCOCH(Cl)-CH₃ | H | -CH(CH₃)₂ |
| MJ1338-RS | H | H | =NNHCOC₆H₄(OH)(2) | H | -CH(CH₃)₂ |
| MJ1366-RS | H | Br | =N-O-CH₂C₆H₄NO₂(4) | H | -CH(CH₃)₂ |
| | | | | | |
|---|---|---|---|---|---|
| * Dihydrobetulinsäure | | | | | |

2. Verwendung eines Betulinsäurederivats oder einer Kombination mehrerer Betulinsäurederivate nach Anspruch 1 zum Herstellen einer Zusammensetzung, die bei der Behandlung eines Patienten mit Angiogenese nützlich ist.

3. Verwendung nach Anspruch 2, wobei die Angiogenese Tumor-assoziiert ist.

4. Verwendung nach Anspruch 3, wobei besagter Patient ein Mensch, ein Säugetier oder ein anderes Tier ist.

5. Verwendung nach Anspruch 3, wobei der ED₅₀-Wert von Betulinsäurederivaten gegen menschliche Nabelschnurvenen-Endothelzellen 0,35 bis 4,0 µg/ml ist.

6. Verwendung nach Anspruch 3, wobei die Endothelzellspezifität von Betulinsäurederivaten für Prostatakrebs 1,04 bis 21,1 ist.

7. Verwendung nach Anspruch 3, wobei die Endothelzellspezifität von Betulinsäurederivaten für Lungenkrebs 0,43 bis > 10 ist.

8. Verwendung nach Anspruch 3, wobei die Endothelzellspezifität von Betulinsäurederivaten für Eierstockkrebs 0,54 bis 7,0 ist.

9. Verwendung nach Anspruch 3, wobei die Endothelzellspezifität von Betulinsäurederivaten für Colonkrebs 0,87 bis 14,3 ist.

10. Verwendung nach Anspruch 3, wobei das Betulinsäurederivat an den Patienten in einem pharmazeutisch annehmbaren Zusatzstoff, Träger, Verdünnungsmittel, Lösungsmittel, Füllstoff, Schmiermittel, Bindemittel, Binder oder Stabilisator verabreicht wird.

11. Verwendung nach Anspruch 3, wobei das Betulinsäurederivat in Form einer Tablette, Pastille, Kapsel, Pulver, wäßriger oder öliger Suspension, Sirup, Elixier, Implantat oder wäßriger Lösung verabreicht wird.

12. Verwendung nach Anspruch 3, wobei die Dosierung für einen Menschen im Bereich von 1,0 bis 200 mg/kg/Tag beträgt.

13. Verwendung nach Anspruch 3, wobei das Betulinsäurederivat oder Derivate oder eine Kombination davon an den Patienten systemisch verabreicht wird.

## Revendications

1. Dérivé d'acide bétulinique, représenté par la formule suivante : 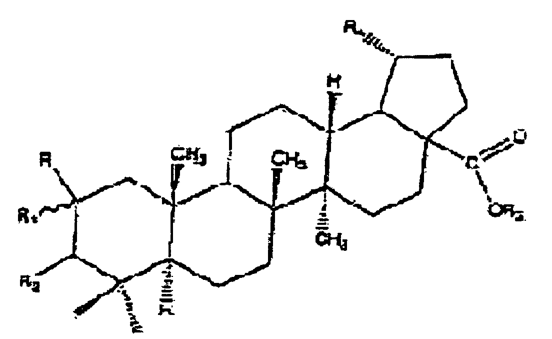 dans laquelle une combinaison de R, R₁, R₂, R₃ et R₄ est choisie dans le groupe comprenant
| DERIVE | R | R₁ | R₂ | R₃ | R₄ |
|---|---|---|---|---|---|
| MJ353-RS | H | H | =NNHC₆H₅ | H | CH₂=CCH₃ |
| MJ548-RS | H | Br | =O | CH₂CH₂COO- CH₃ | BrCH₂-C(Br)CH₃ |
| MJ-878-RS | H | H | -NHNH C₆H₅ | H | -CH(CH₃)₂ |
| MJ-912-RS | H | H | -NHNH C₆H₄ (OCH₃) [4] | H | -CH(CH₃)₂ |
| MJ-935-RS | H | H | -OCO-C₆H₃F₂[3,5] | H | -CH(CH₃)₂ |
| MJ-937-RS | H | H | -OCO-C₆H₃F₂[2,4] | H | -CH(CH₃)₂ |
| MJ939-RS | H | H | -OCOC₆H₄(CF₃) [3] | H | CH₂=C-CH₃ |
| MJ943-RS | H | H | -OCOC₆H₄ (CF₃) [2] | H | -CH (CH₃)₂ |
| MJ998-RS | H | H | -N=CHC₆H₃F₂ [3,4] | H | -CH (CH₃)₂ |
| MJ1065-RS | H | H | -N=CHC₆H₃F₂[(2,4) | H | -CH(CH₃)₂ |
| MJ1098-RS | H | H | NOCH₂C₆H₄NO₂(4) | H | -CH(CH₃)₂ |
| MJ1101-RS | H | H | -OH | COCH₂=CH₂ | CH₂=C-CH₃ |
| MJ1103-RS | H | H | -OH | COCH₂=CH₂ | -CH (CH₃)₂ |
| MJ1104-RS | H | H | -OCOC₆H₄ (C₅H₁₁) [4] | H | CH₂=C-CH₃ |
| MJ1108-RS | H | H | -OCOCH₂C₆H₃(OCH₃)₂[2,5] | H | CH₂=C-CH₃ |
| MJ1138-RS | H | H | -OCOC₆H₄ (C₇H₁₅) [4] | H | CH₂=C-CH₃ |
| MJ1161-RS | H | H | -OH | 3-désoxy DHBA * | -CH (CH₃)₂ |
| MJ1183-RS | H | H | -OCOCH₂C₆H₃(OCH₃)₂[3,4] | H | CH₂=C-CH₃ |
| MJ1204-RS | H | H | =O | COCH=CH₂ | -CH(CH₃)₂ |
| MJ1205-RS | H | H | -OCO C₆F₅ | H | CH₂=C-CH₃ |
| MJ1210-RS | H | H | =NOH | COCH=CH₂ | -CH(CH₃)₂ |
| MJ1213-RS | H | H | -OCO C₆F₅ | H | -CH(CH₃)₂ |
| MJ1283-RS | H | H | -OCOC₆H₃(OCH₃)₂[3,4] | H | -CH (CH₃)₂ |
| MJ1287-RS | H | H | -OCOC₆H₃(OCH₃)₂[2,4] | H | -CH (CH₃)₂ |
| MJ1295-RS | H | H | -OCOCClF₂ | H | -CH (CH₃)₂ |
| MJ1296-RS | H | H | -OCOC₆H₅-C₆H₅ | H | CH₂=C-CH₃ |
| MJ1298-RS | H | H | -OCOCH(Cl)Ph | H | CH₂=C-CH₃ |
| MJ1301-RS | H | H | -OCO- (CH₂)₃COOH | H | -CH (CH₃)₂ |
| MJ1304-RS | H | H | -OCOC₆H₄Cl [4] | H | CH₂=C-CH₃ |
| MJ1305-RS | H | H | -OCOC₆H₄Cl [4] | H | -CH (CH₃)₂ |
| MJ1315-RS | H | H | -OCOC₆H₄(CHCl₂) (3) | H | CH₂=C-CH₃ |
| MJ1316-RS | H | H | -OCOC₆H₄(CHCl₂) (3) | H | -CH (CH₃)₂ |
| MJ1312-RS | H | H | -OSO₂CH₂CH₂CH₂Cl | H | CH₂=C-CH₃ |
| MJ1313-RS | H | H | -OSO₂CH₂CH₂CH₂Cl | H | -CH(CH₃)₂ |
| MJ1327-RS | H | H | -OCOC₆H₂(COOH)(2)Cl₂(3,6) | H | CH₂=C-CH₃ |
| MJ1328-RS | H | H | -OCOC₆H₂(COOH) (2)Cl₂(3,6) | H | -CH(CH₃)₂ |
| MJ1335-RS | H | H | -OCOCH(Cl)-CH₃ | H | CH₂=C-CH₃ |
| MJ1336-RS | H | H | -OCOCH(Cl)-CH₃ | H | -CH(CH₃)₂ |
| MJ1338-RS | H | H | =NNHCOC₆H₄(OH) (2) | H | -CH (CH₃)₂ |
| MJ1366-RS | H | Br | =N-O-CH₂C₆H₄NO₂(4) | H | -CH (CH₃)₂ |
| | | | | | |
|---|---|---|---|---|---|
| * *Acide dihydrobétulinique* | | | | | |

2. Utilisation d'un dérivé d'acide bétulinique ou d'une combinaison de plusieurs dérivés d'acide bétulinique selon la revendication 1 pour fabriquer une composition utile dans le traitement d'un patient avec angiogenèse.

3. Utilisation selon la revendication 2, dans laquelle l'angiogenèse est associée à une tumeur.

4. Utilisation comme revendiqué dans la revendication 3 dans laquelle ledit patient est un homme, un mammifère ou un autre animal.

5. Utilisation comme revendiqué dans la revendication 3 dans laquelle la valeur DE₅₀ de dérivés d'acide bétulinique contre les cellules endothéliales de la veine ombilicale humaine est de 0,35 à 4,0 µg/ml.

6. Utilisation comme revendiqué dans la revendication 3 dans laquelle la spécificité envers les cellules endothéliales de dérivés d'acide bétulinique pour le cancer de la prostate est de 1,04 à 21,1.

7. Utilisation comme revendiqué dans la revendication 3 dans laquelle la spécificité envers les cellules endothéliales de dérivés d'acide bétulinique pour le cancer du poumon est de 0,43 à > 10.

8. Utilisation comme revendiqué dans la revendication 3 dans laquelle la spécificité envers les cellules endothéliales de dérivés d'acide bétulinique pour le cancer de l'ovaire est de 0,54 à 7,0.

9. Utilisation comme revendiqué dans la revendication 3 dans laquelle la spécificité envers les cellules endothéliales de dérivés d'acide bétulinique pour le cancer du colon est de 0,87 à 14,3.

10. Utilisation comme revendiqué dans la revendication 3 dans laquelle le dérivé d'acide bétulinique est administré au patient dans un additif, vecteur, diluant, solvant, charge, lubrifiant, excipient, liant ou stabilisant pharmaceutiquement acceptable.

11. Utilisation comme revendiqué dans la revendication 3 dans laquelle le dérivé d'acide bétulinique est administré sous forme de comprimé, pastille, capsule, poudre, suspension aqueuse ou huileuse, sirop, élixir, implant ou solution aqueuse.

12. Utilisation comme revendiqué dans la revendication 3 dans laquelle le dosage pour un homme est dans la fourchette de 1,0 à 200 mg/kg/jour.

13. Utilisation comme revendiqué dans la revendication 3 dans laquelle le ou les dérivés d'acide bétulinique ou une combinaison de ceux-ci sont administrés au patient de manière systémique.
